# EUROPEAN PATENT SPECIFICATION

(11) **EP 2 367 791 B1**
(45) Date of publication and mention of the grant of the patent: **19.09.2012**
(21) Application number: 10728525.6
(22) Date of filing: 21.06.2010
(51) Int. Cl.: C07D 209/42, A61K 31/404, A61P 9/12

(54) **PERINDOPRIL TOSYLATE**
PERINDOPRILTOSYLAT
TOSYLATE DE PÉRINDOPRIL

(30) Priority: 20.06.2009 GB 0910692
(43) Date of publication of application: 28.09.2011
(73) Proprietor: Teva Pharmaceutical Industries Ltd., 49131 Petah Tiqva (IL)
(72) Inventor: COSTI, Sonia, Rosh Hayin (IL); KHAN, Mubeen, Maharashtra 400 703 (IN); GHARPURE, Milind, Maharashtra State 400709 (IN); PANDEY, Anand, Kumar, Dist Pratapgarh UP-230128 (IN); YADAV, Roop Singh, Nearest city-Jhansi U.P -210429 (IN); PATIL, Pramod Vittal, District Raigad 410206 (IN); LAD, Sachin, Thane 401105 (IN); PATLE, Girish, Thane-421501 (IN)
(74) Representative: Gallagher, Kirk James
(86) International application number: PCT/US2010/039350
(87) International publication number: WO 2010/148396

(56) References cited:
- EP-A1- 1 354 873
- WO-A1-2007/017087

## Description

### BACKGROUND OF THE INVENTION

### 1. Technical Field

The present invention relates to a novel salt of perindopril, namely the paratoluene sulfonic acid salt.

### 2. Description of the Related Art

Perindopril and its pharmaceutically acceptable salts are known as angiontensin converting enzyme inhibitors and are used in the treatment of cardiovascular diseases, especially in the treatment of hypertension and heart failure. Perindopril tertiary butyl amine (perindopril erbumine) is an approved salt and is available in the market as tablets under the brand name ACEON^{®} in the dosage strengths of 2 mg, 4 mg and 8 mg for oral administration.

Perindopril erbumine is chemically described as 2S,3aS,7aS)-1-[(S)-N-[(S)-1-carboxy-butyl]alanyl]hexahydro-2-indolinecarboxylic acid, 1-ethyl ester, compound with tert-butylamine (1:1) and can be represented by structural formula:

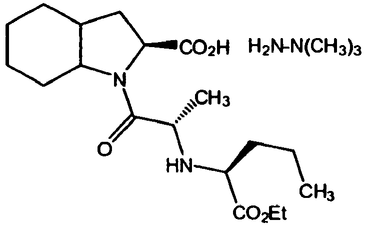

U.S. Patent No. 4,508,729 and E.P. Patent No. 0049658 B1 describe perindopril optically pure isomer and in the form of sodium salt, a process for the preparation thereof, a pharmaceutical composition and a method of use. U.S. Patent No. 6696481B2 and E.P. 1354873B1 describe an arginine salt of perindopril and process for its preparation, a pharmaceutical composition and a method of use. U.S. publication No. 20080139823 A1 and International publication WO006097941 describe a dicyclohexyl amine salt of perindopril and a process for its preparation, a pharmaceutical composition and a method of use. U.S. publication No. 20080306135A1 and E.P. publication No. 1910287A1 describes tertiary octyl amine and neo pentyl amine salts of perindopril and processes for their preparation, a pharmaceutical composition and a method of use.

In view of the intrinsic fragility of perindopril, increasingly stable salt forms are required.

It has been found that the tosylate salt of perindopril has certain advantages over other salts reported earlier. The synthesis of perindopril tosylate is also simple, ecofriendly, robust, and well suited on commercial scale.

### SUMMARY OF THE INVENTION

In one aspect the present invention comprises perindopril tosylate. Perindopril tosylate is chemically described as 2S,3aS,7aS)-1-[(S)-N-[(S)-1-carboxybutyl]alanyl]hexahydro-2-indolinecarboxylic acid, 1-ethyl ester, compound with paratoluene sulfonic acid (1:1) and can be represented by structural formula:

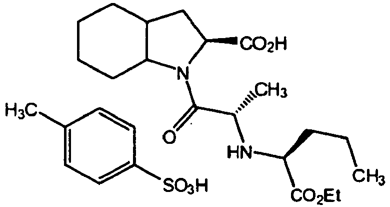

In a further aspect, the present invention provides a pharmaceutical composition comprising perindopril tosylate, and at least a pharmaceutically acceptable carrier.

In a still further aspect the present invention comprises a method of treating hypertension and/or heart failure which comprises administering a therapeutically effective amount of perindopril tosylate.

In one embodiment, the present invention refers to the use of the above described perindopril tosylate for the preparation of a formulation.

In yet another embodiment, the present invention refers to a pharmaceutical composition comprising perindopril tosylate and at least one pharmaceutically acceptable excipient.

### BRIEF DESCRIPTION OF THE ACCOMPANYING FIGURES

Fig. 1: Shows the ¹H NMR spectrum of Perindopril tosylate prepared according to example 2.

Fig 2: Shows a proton NMR (D₂O-Exchange) spectrum of Perindopril tosylate prepared according to example 2.

Fig. 3: Shows a MASS spectrum of Perindopril tosylate prepared according to example 2.

Fig. 4: Shows a FTIR spectrum of Perindopril tosylate prepared according to example 2.

Fig. 5: Shows an X-ray powder diffraction pattern of Perindopril tosylate prepared according to example 2.

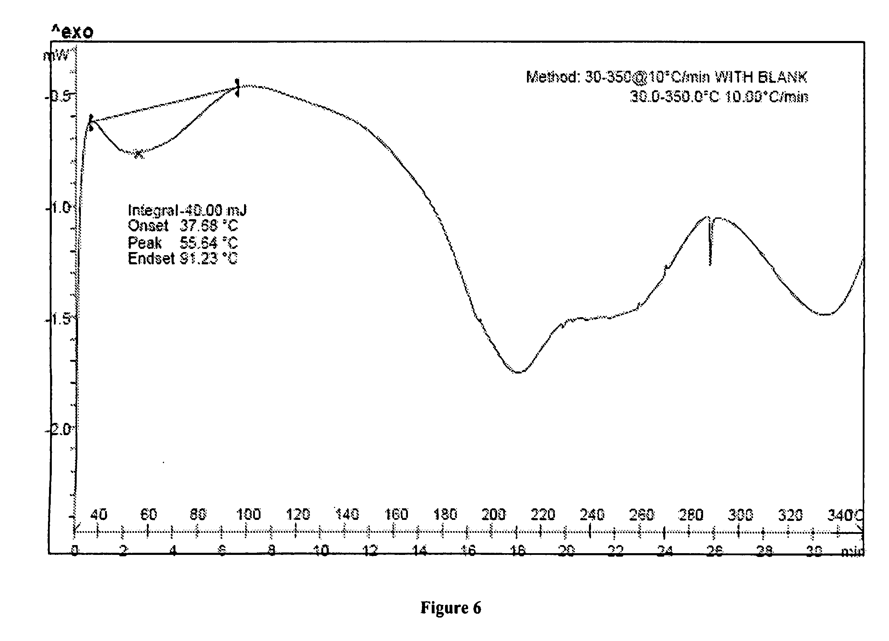

Fig. 6: Differential scanning calorimetry of Perindopril tosylate prepared according to example 2.

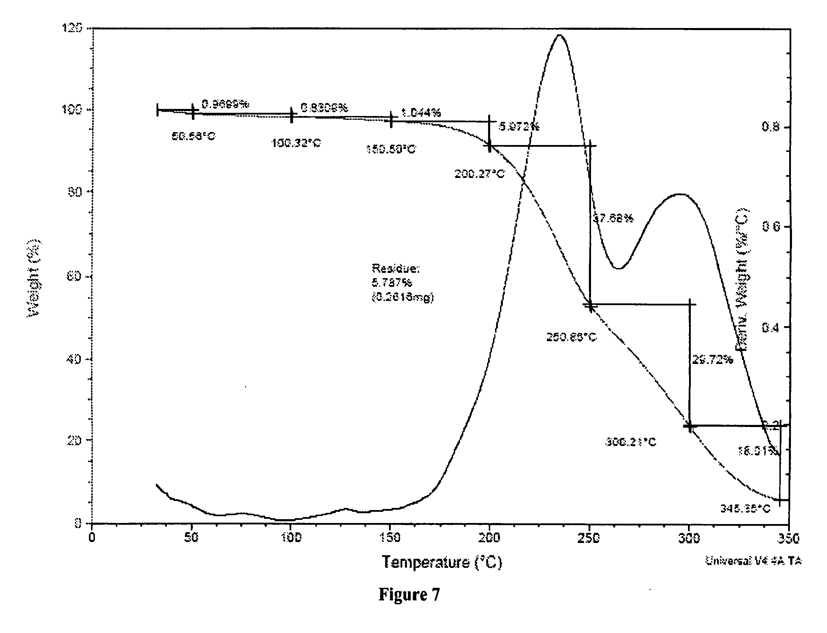

Fig. 7: Shows a Thermo gravimetric analysis thermogram of Perindopril tosylate prepared according to example 2.

Fig. 8: Shows a solid state ¹³C NMR spectrum of Perindopril tosylate

Fig. 9: Shows a solid state ¹³C NMR spectrum of Perindopril tosylate

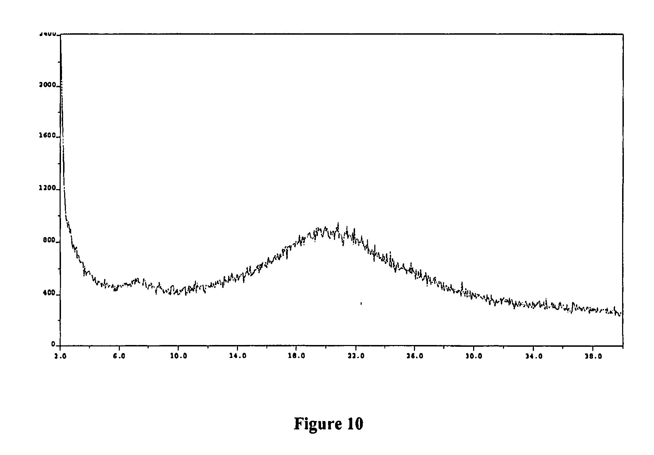

Fig. 10: Shows an X-ray powder diffraction pattern of Perindopril tosylate prepared according to example 10.

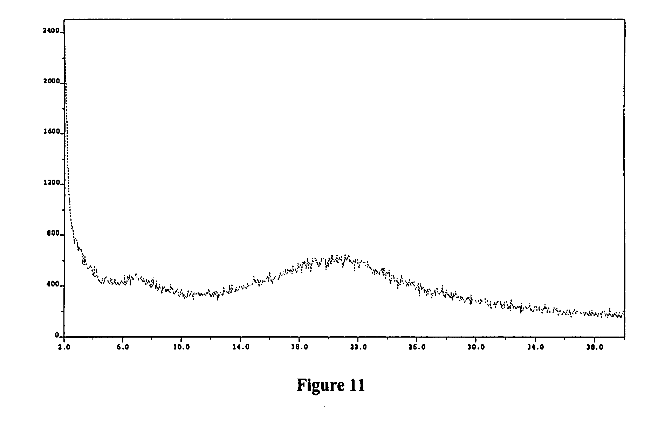

Fig. 11: Shows an X-ray powder diffraction pattern of Perindopril tosylate prepared according to example 11.

### DETAILED DESCRIPTION OF THE INVENTION

The present invention relates to the tosylate salt of perindopril. The present invention also relates to processes for their preparation, pharmaceutical compositions comprising them and their use in medicine, particularly in treating hypertension and heart failure.

Any matter, e.g., a reaction mixture, may be characterized herein, unless otherwise noted, as being at, or allowed to come to "room temperature." This expression means that the temperature of the thing is close to, or the same as, that of the space, *e.g*., the room or fume hood, in which the thing is located. Typically, room temperature is from about 20° C to about 30° C, or about 25° C.

A process or portion thereof may be referred to herein as being carried out "overnight." This term refers to a time interval, *e.g*., for carrying out the process or portion thereof, that spans the time during the night, when that process or step may not be actively observed. This time interval is from about 8 to about 20 hours, typically about 16 hours.

In one aspect, the present invention provides a tosylate salt of perindopril.

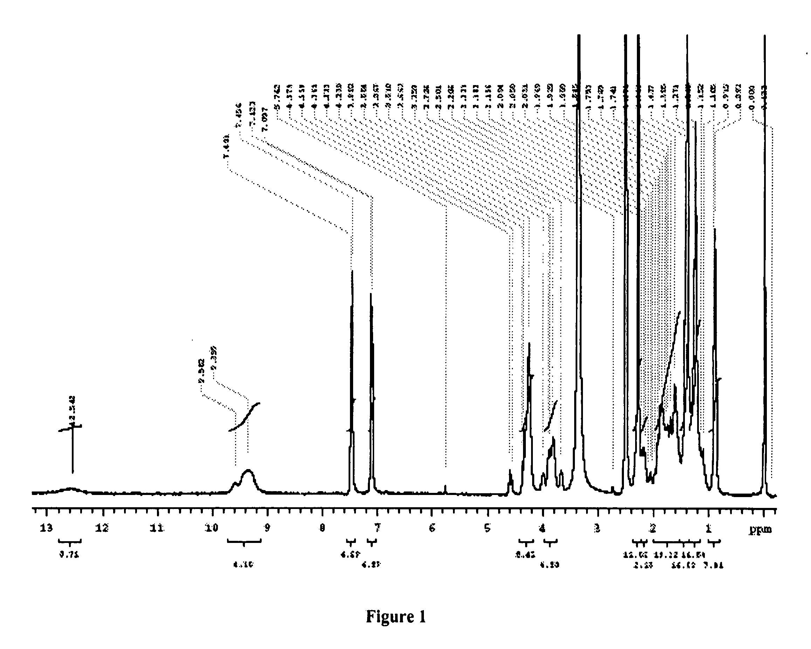

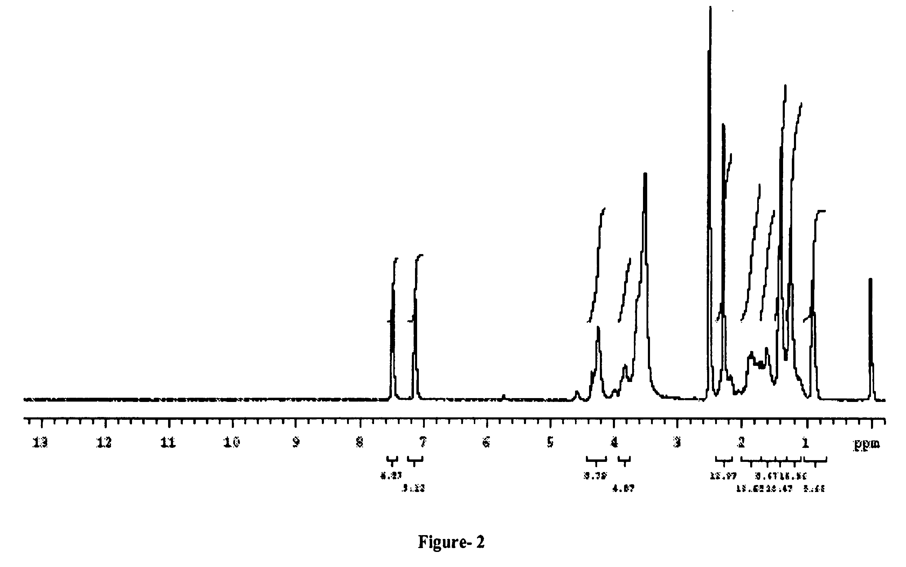

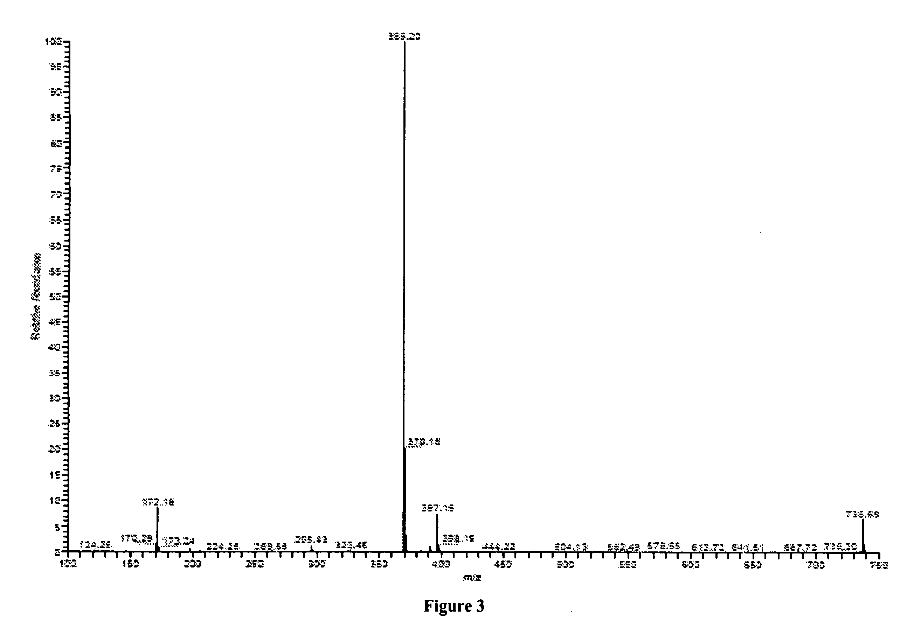

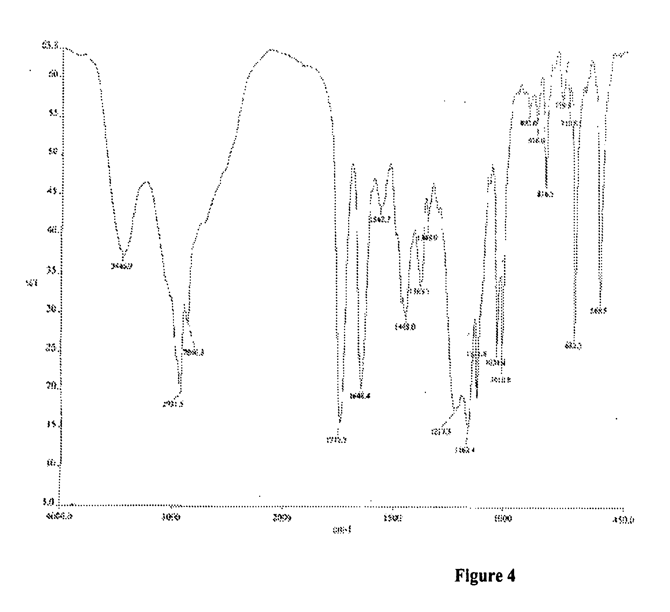

The tosylate salt of perindopril may be characterized by ¹H NMR spectrum substantially as illustrated in Figure 1. The tosylate salt of perindopril may further be characterized by proton NMR (D₂O-Exchange) spectrum substantially as illustrated in Figure 2. The tosylate salt of perindopril may further be characterized by a mass (M/S) spectrum substantially as illustrated in Figure 3 The tosylate salt of perindopril may further be characterized by FTIR spectrum substantially as illustrated in Figure 4.

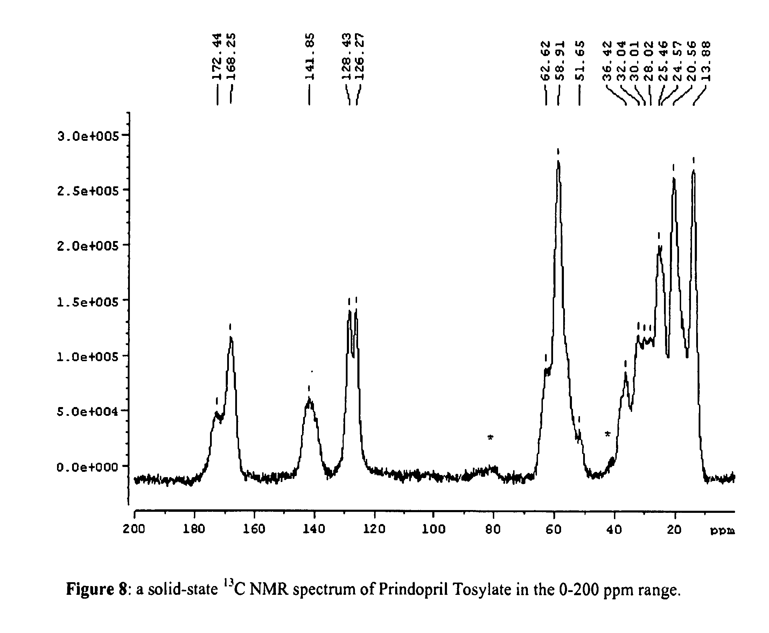

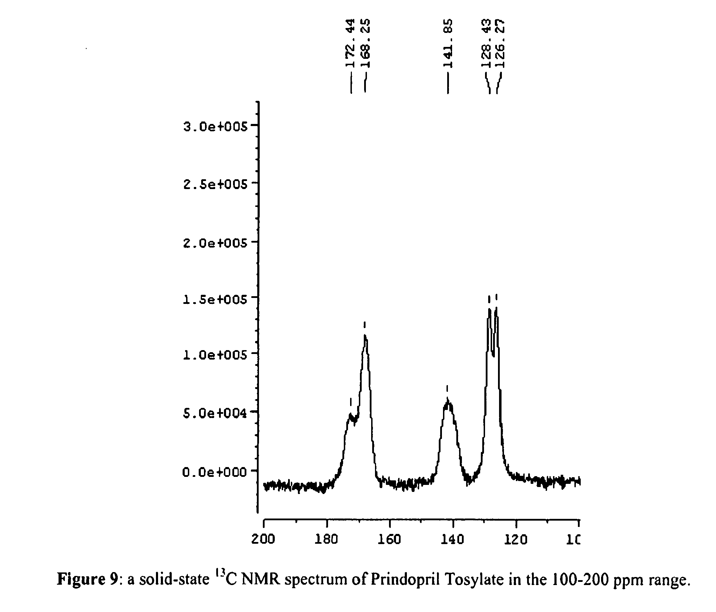

The present invention provides Perindopril tosylate characterized by solid-state ¹³C NMR spectrum with signals at about 126.3, 128.4 and 168.3 ± 0.2 ppm; a solid-state ¹³ C NMR spectrum having chemical shifts differences between the signal exhibiting the lowest chemical shift and another in the chemical shift range of 100 to 180 ppm of about 0.0, 2.1 and 42.0 ± 0.1 ppm, wherein the signal exhibiting the lowest chemical shift in the chemical shift area of 100 to 180 ppm is typically at about 126.3 ± 1ppm. A ¹³C NMR spectrum for Prindopril tosylate is depicted in Figures 8 and 9.

Perindopril tosylate has a spherical shape. It contains a narrow distribution of prime particles together with aggregates of great number of small particles. The particle size of the spherical material, analyzed with microscope observation is up to 10-15 mcm. Particle shape and uniformity can have major influence on dissolution rate. Unified and smaller particles may be preferred because of that.

In yet another aspect, the present invention provides a process for preparing perindopril tosylate comprising:
(a) providing a solution of perindopril and paratoluene sulfonic acid in one or more reaction solvents;
(b) removing the reaction solvent(s) from the solution; and
(c) recovering the perindopril tosylate.

The solution of perindopril and paratoluene sulfonic acid can be obtained by dissolving perindopril and paratoluene sulfonic acid in a solvent either by agitation at room temperature or at elevated temperatures.

As used herein, a reaction solvent is any liquid substance capable of dissolving perindopril and paratoluene sulfonic acid. The reaction solvent can include, but is not limited to, water, alcohols such as methanol, ethanol, n-propanol, isopropanol, n-butanol, isobutanol, and tertiary butyl alcohol and the like; ketonic solvents such as acetone, methyl ethyl ketone, methyl isobutyl ketone, 2-butanone and the like; chlorinated solvents such as methylene chloride; ester solvents such as ethyl acetate, aromatic hydrocarbon such as toluene, nitrile solvents such as acetonitrile, propionitrile and the like; or mixtures thereof or their aqueous mixtures in various proportions without limitation. Preferably, the solvent is selected from alcohols, ketones, nitriles and their aqueous mixtures and water.

The volume of the reaction solvent used to solubilize perindopril and paratoluene sulfonic acid can range from about 2 to about 20 volumes to the weight of the perindopril free acid taken, preferably from about 3 to about 15 volumes.

The molar ratio of paratoluene sulfonic acid to perindopril is preferably from about 1:0.8 to about 1:1.2, most preferably about 1:1.

The temperature for obtaining a clear and homogenous solution can range from about 25°C to about 75°C or to about the boiling point of the reaction solvent/s used, preferably from about 25°C to about 40°C.

The reaction solution obtained is optionally filtered through diatomaceous earth (e.g., celite) to separate the extraneous matter present or formed in the solution by using conventional filtration techniques known in the art.

The immiscible organic solvents that can be used to extract the compound formed include, but are not limited to, water immiscible solvents like halogenated solvent such as dichloromethane, ethylene dichloride, chloroform and the like; esters such as ethyl acetate, isopropyl acetate and the like; hydrocarbons such as n-hexane, n-heptane, cyclohexane, toluene and the like; and mixtures thereof. One preferred solvent is dichloromethane.

Removal of the solvent can be performed using spray drying or lyophilization. Spray drying broadly refers to processes involving breaking up liquid mixtures into small droplets (atomization) and rapidly removing solvent from the mixture. In a typical spray drying apparatus, there is a strong driving force for evaporation of solvent from the droplets, which may be provided by providing a drying gas. Spray drying processes and equipment are described in Perry's Chemical Engineer's Handbook, pp. 20-54 to 20-57 (6th ed. 1984) and Remington: The Science and Practice of Pharmacy, 19th ed., vol. II, pg. 1627.

By way of non-limiting example only, the typical spray drying apparatus comprises a drying chamber, atomizing means for atomizing a solvent-containing feed into the drying chamber, a source of drying gas that flows into the drying chamber to remove solvent from the atomized-solvent-containing feed, an outlet for the products of drying, and product collection means located downstream of the drying chamber. Examples of such apparatuses include Niro Models PSD-1, PSD-2 and PSD-4 (Niro A/S, Soeborg, Denmark). Typically, the product collection means includes a cyclone connected to the drying apparatus. In the cyclone, the particles produced during spray drying are separated from the drying gas and evaporated solvent, allowing the particles to be collected. A filter may also be used to separate and collect the particles produced by spray drying.

The processes of the present invention may preferably employ spray drying with an inlet temperature of from about 50°C to about 200°C. Most preferably the inlet temperature is at about 60°C to about 120°C. The spray drying may preferably be conducted with a solution feed rate of less than about 5 L/h, more preferably about 1 L/h.

The spray drying may preferably be conducted with an outlet temperature of below the inlet temperature, more preferably below about 100°C, and most preferably about 65°C. The drying gas used in the process of the present invention may be any suitable gas, although inert gases such as nitrogen, nitrogen-enriched air, and argon are preferred.

The lyophilization is done by a process comprising cooling the solution to obtain a cooled mixture, and evaporating the solvent while maintaining the mixture cooled at low temperatures.

Preferably, the solution is cooled to a temperature below ambient temperature, for example to about 0°C, and then further to about -40°C providing the cooled mixture,which is a frozen mass.

Typically, the frozen mass is then subjected to a pressure of less than about one atmosphere, to remove the solvent.

Typically, the solution is provided by combining perindopril free acid, water and para toluene sulfonic acid and stirring the combination; optionally the combination can be washed with a water immiscible organic solvent, for example toluene, hexane, dichloromethane, ethyl acetate, methyl ethyl ketone etc.

Removal of the reaction solvent and/or water immiscible organic solvent can also be accomplished by, for example, substantially complete evaporation of the solvent, concentrating the solution. Alternatively, the solvent may also be removed by evaporation. The solution may also be evaporated (completely or substantially) in, for example, a pilot plant Rota vapor, a Vacuum Paddle Dryer or in a conventional reactor under vacuum above about 720 mm Hg. Preferably, the removal of solvent(s) is by evaporation by distillation under vacuum.

In one embodiment, spray drying techniques can be used to remove the solvent(s), in which a solution of perindopril or its salt is sprayed into the spray drier at the flow rate ranging from about 10 ml/hr to about 300 ml/hr, preferably at flow rate of about 40 ml/hr to about 200ml/hr. The air inlet temperature to the spray drier used may range from about 25°C to about 150°C, preferably from about 60°C to about 110°C and the outlet air temperature used may range from about 30°C to about 90°C preferably from about 35°C to about 50°C.

In one embodiment, agitated thin film evaporation technology can also be used for removal of the solvent(s), which involves separating the volatile component using indirect heat transfer coupled with mechanical agitation of the flowing film under controlled conditions. In vertical agitated thin-film drying (or evaporation) (ATFD-V), the starting solution is fed from the top into a cylindrical space between a centered rotary agitator and an outside heating jacket. The rotor rotation agitates the downside-flowing solution while the heating jacket heats it.

Recovery of the perindopril tosylate obtained in the above processes, can be performed by any conventional method, such as filtration, decantation and centrifugation, known in the art.

Preferably, recovery comprises filtering, washing, and drying the solid. Washing is usually done with the same solvent used in the reaction.

The perindopril used as starting material in the processes described herein above, may be of indefinite morphology, i.e. crystalline or amorphous or any mixture thereof, or even a crude perindopril resulting from synthetic processing step, including synthetic processing steps known in the art. Illustratively, US. Patent Nos. 4914214 and 7521566 are mentioned.

In a specific embodiment, the process first comprises preparation of starting material perindopril free acid used herein by neutralizing a perindopril salt with an acid. Suitable acids include but are not limited to mineral acids, such as hydrochloric acid, p-toluene solfonic acid, hydrobromic acid, sulfuric acid and the like. The most preferred acid is hydrochloric acid. The perindopril salt that can be used as starting material for making perindopril free acid include but are not limited to perindopril erbumine, sodium, HCl, arginine, dicyclohexyl amine, tertiary octylammonium salt or perindopril neopentylamine salt, preferably the salt is perindopril erbumine.

Preferably after combining of the salt and the acid, the reaction mixture is then stirred for about 15 minutes to about 1.5 hours, preferably for about an hour. Preferably, the reaction mixture is stirred at a temperature of from about 10°C to about 55°C, more preferably at room temperature. Optionally, sodium chloride can be added and the reaction mixture is further stirred for about 15 minutes.

A water immiscible organic solvent is added to the reaction mixture, preferably after neutralization. Examples of such organic solvents include C₃ to C₇ esters, C₁ to C₃ chlorinated hydrocarbons, C₆ to C₁₀ aromatic hydrocarbons or mixtures thereof. Specific examples of such solvents include ethyl acetate, dichloromethane, toluene, chloroform or mixtures thereof. Preferably dichloromethane or a mixture of toluene and dichloromethane.

If an aqueous solution of acid is used with a water immiscible organic solvent, the reaction mixture will form aqueous and organic layers. The layers are preferably separated. The combined organic layers can be heated at a temperature of from about 15°C to about 55°C, more preferably at a temperature of about 35°C.

Recovery of perindopril free acid can be achieved by concentrating the reaction mixture, e.g., if the reaction mixture is too dilute. Concentration can be achieved by removing the solvent, preferably by evaporation. Evaporation can be carried out under a pressure of less than one atmosphere, or less than 700mmHg. A rotary evaporator can be used. If crystallization does not occur, additional solvent can be added, and the inner surfaces of the vessels of the reaction mixture can be scratched. The resulting precipitate can be separated from the reaction mixture by filtration.

The recovered crystals can be dried. Drying can be carried out under a pressure of less than one atmosphere, or less than 760 mmHg, most preferably less than 25mmHg at about 25°C to about 35°C for about 12 hours to about 24 hours.

Optionally, the processes described above can be carried out in one pot.

The characterization of the amorphous perindopril tosylate is analyzed by X-ray powder diffraction were performed on a Philips X'pert PRO Diffractometer using Cu Kα radiation (Cu Kα1 = 1.54060Å). The X-ray source is operated at 45 kV and 40mA. Spectra are recorded in the 2θ range of 2-50°, a step size 0.0167° with a "time-per-step" optimized to 1000 seconds.

The present invention provides perindopril tosylate obtained by the above described processes having a purity, as measured by HPLC, of at least about 98%, more preferably, at least about 99% and most preferably at least about 99.5%.

Preferably, the perindopril tosylate of the invention is substantially pure such that the chemical purity of the perindopril tosylate is about 99% or more, more preferably about 99.5% or more, more preferably about 99.8% or more, more preferably about 99.9% or more, as measured by area under HPLC.

Preferably, the perindopril tosylate is at least 70% amorphous, preferable at least 80% amorphous, more preferable at least 90% amorphous, even more preferable at least 95% amorphous, most preferably at least 99% amorphous.

In one embodiment, the present invention provides perindopril tosylate having less than about 0.20% of any single impurity as measured by area under HPLC peaks. Preferably, the perindopril or a pharmaceutically acceptable salt thereof has less than about 0.15%, more preferably 0.1 %, of any single chemical impurity as measured by area under HPLC peaks.

Perindopril tosylate obtained by the process described above, comprises less residual organic solvents or organic volatile impurities than the amount recommended for pharmaceutical products, as set forth for example in ICH guidelines and U.S. pharmacopoeia; e.g., less than about 600ppm of dichloromethane, less than about 5000 ppm of ethanol, acetone, ethyl acetate, isopropyl alcohol, less than about 890 ppm of toluene and less than about 290 ppm of n-hexane.

The unformulated perindopril tosylate salt obtained by the process of present invention that is used as starting material in preparing a pharmaceutical composition generally has a D₉₀ particle size of less than 400 microns; preferably less than about 200 microns; more preferably less than 150 microns; still more preferably less than about 50 microns; and still more preferably less than about 15 microns.

Any milling, grinding micronizing or other particle size reduction method known in the art can be used to bring the perindopril tosylate into any desired particle size range as set forth above.

Also described herein is amorphous form of perindopril tosylate.

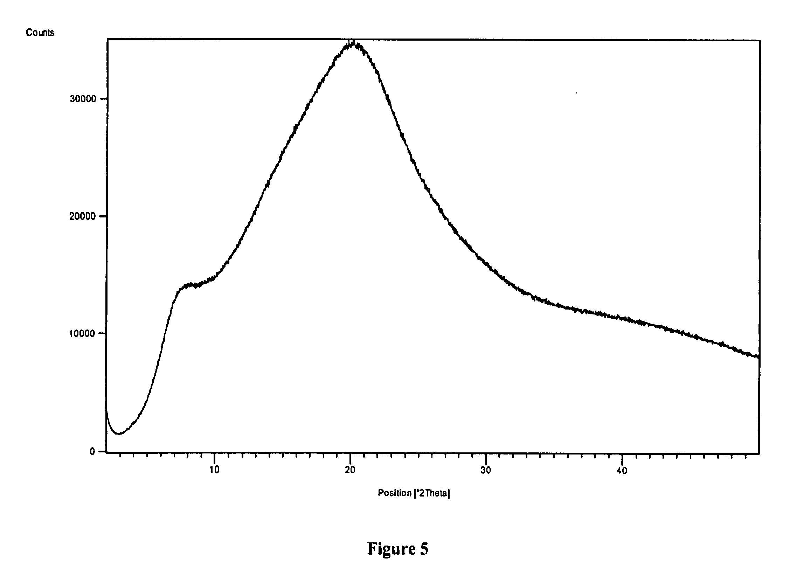

The amorphous form of perindopril tosylate may be characterized by powder XRD pattern substantially as illustrated in Figure 5.

The perindopril tosylate substantially in an amorphous form obtained by the above processes may be further dried in, for example, vacuum tray dryer, rotocon vacuum dryer, vacuum paddle dryer or pilot plant rotavapor, to further lower residual solvents. When implemented, the preferred instrument is a vacuum tray dryer. The temperature for drying can range from about 25°C to about 55°C, preferably from about 25°C to about 40°C under vacuum. The drying can be carried out for any desired time, time periods can range from 1 to 20 hours, preferably 5 to 10 hours.

The above described form of perindopril tosylate can be used to prepare formulations by any method known in the art.

In another embodiment, the present invention provides a pharmaceutical composition comprising perindopril tosylate obtained by the processes described herein, and at least a pharmaceutically acceptable carrier.

The process for the preparation of perindopril tosylate of the present invention is eco-friendly and easily scaleable.

The following examples are provided to enable one skilled in the art to practice the invention and are merely illustrative of the invention. The examples should not be read as limiting the scope of the invention as defined in the features and advantages.

### EXAMPLES

### A. Instrumentation and methods

### Powder X-ray diffraction:

### Incident Beam Optics :

| | |
|---|---|
| PreFIX Module | : Programmable Divergence Slit and Anti-scatter Slit (Offset 0.00°) |
| Filter | : Nickel |
| Soller Slit | : Soller 0.02 rad |
| Mask | : 10mm |
| Divergence Slit | : PDS Automatic, 10mm Irradiated length, Offset 0.00mm |
| Anti-scatter Slit | : Slit Fixed 1/2° |
| Beam Knife | : Beam Knife for MPD systems |

### Diffracted Beam Optics:

| | |
|---|---|
| PreFIX Module | : X'Celerator (Offset 0.00°) |
| Soller Slit | : Soller 0.02 rad |
| Anti-scatter Slit | : Programmable Anti-scatter Slit Automatic, 10mm Observed length, Offset 0.00mm |
| Detector | : X'Celerator[1] Scanning Mode, Active length (2Theta) = 2.122° |

### Measurement Parameters:

| | |
|---|---|
| Scan axis | : Gonio |
| Scan mode | : Continuous |
| Start angle (°) | : **2.0** |
| End angle (°) | : **50.0** |
| Step size (°) | : 0.0167 |
| Time per step (s) | : **50.0** |

### Sample Preparation:

Take adequate amount of sample as to fill the sample holder using Back-loading technique. Then the sample holder was loaded between the X-ray optics-path and scanned using the above-described parameters. Obtained powder X-ray diffraction profiles were integrated using X'Pert HighScore Software.

### ¹H NMR Spectroscopy: -

¹H NMR spectrum was recorded in CDCL3 using 300 MHz Varian NMR spectrometer.

### Sample Preparation for 1H NMR:-

Take 5mg sample in NMR tube and dissolved in 0.7ml CDCL3.

### ¹³C NMR Spectroscopy: -

¹³C NMR spectrum was recorded in CDCL3 using 300 MHz Varian NMR spectrometer.

### Sample Preparation for ¹³C NMR: -

Take 45mg sample in NMR tube and dissolved in 0.7ml CDCL3

### i. ¹³C NMR

¹³C NMR at 125MHz using Bruker Avance II+ 500
SB probe using 4mm rotors
Magic angle was set using KBr
Homogeneity of magnetic field checked using adamantane
Parameters for Cross polarization optimized using glycine
Spectral reference set according to glycine as external standard (176.03 ppm for low field carboxyl signal)

### Scanning parameters:

Magic Angle Spinning Rate: 11 kHz
Pulse Program: cp with tppm15 during decoupling
Contact time: 2ms
Delay time: 2s
Number of Scans: 2048

### ii. Differential scanning calorimetry (DSC)

Approximately 1-2mg sample was accurately weighed into an Aluminum DSC pan with pin hole lid and the sample was placed into the Mettler Toledo DSC822^{e} equipped with a nitrogen cooling unit and allowed to equilibrate at 30°C until the stable heat flow reference was seen. A dry nitrogen purge gas at a flow rate of 50ml/minute was used to produce inert atmosphere to prevent oxidation of sample during the heating. The sample was scanned from 30-350°C at rate of 10°C/min and resulting heat flow response was measured against temperature.

### iii. Thermo gravimetric analysis (TGA)

TGA analysis was recorded on TGA Q500 V6.5. Thermogram was recorded at 30-350°C at the rate of 10°C/min. Nitrogen gas flow was 60ml/min.

### iv. Mass spectrometry

Prepared a known concentration solution of Perindopril Tosylate in methanol and monitored using ESI source with positive mode.

### v. Fourier Transform Infrared (FTIR) Analysis

Weigh accurately about 200mg of KBr, previously dried at 200°C and cooled, into a mortar and grind to a fine powder. Add 5.0mg of test sample mix well and grind to a fine powder. Take a small quantity of powder and make a thin semitransparent pellet. Record the IR spectrum of the pellet from 4000cm⁻¹ to 450cm⁻¹ taking air as a reference.

### B. Working Examples

### Example 1: Preparation of Perindopril Free acid

Perindopril erbumine (250.0 g, 0.566 moles) was added to 750 ml of purified water, and the resulting mixture was cooled to about 10-15°C. Hydrochloric acid (113 ml, 50 %v/v) solution was added to the reaction mass at about 10-15°C. Dichloromethane (1250 ml) was added and the resulting mixture was heated to about 25-30°C. The resultant reaction mass was stirred for 30 min. at 25-30°C. The organic and aqueous layers were separated and the aqueous layer was extracted with 750 ml & 375 ml portions of dichloromethane (DCM). All the organic layers were combined and dried over 25.0 g of anhydrous sodium sulphate. The solvent was distilled completely at about 25 to 30°C under vacuum. n-Hexane (250 ml) was added to the resulting residue and this mixture was concentrated. n-Hexane (2.0 L) was charged and this mixture was stirred for 2 hrs at 25-30°C under nitrogen. A solid separated and was filtered. The filtered solid was washed with 250 ml of n-hexane under a nitrogen atmosphere. The solid obtained was dried at about 30-35°C under vacuum for about 12 hrs to afford 166 g of the title compound. % Yield: 80%w/w. Purity by HPLC: 99.8 % All known and Unknown impurities = below 0.1 %.

### Example 2: Preparation of Perindopril Tosylate Using Water

Perindopril free acid (25 g) obtained in example 1, p-toluene sulfonic acid (13 g) and 112.5 ml of water were charged into a clean and dry 4-neck round bottom flask (RBF) and this mixture was stirred for about 3-4 hrs. Sodium chloride (16.8 g) and 125 ml of DCM were charged followed by stirring for about 25-30 min. The organic and aqueous layers were separated and the aqueous layer was extracted with 2x75 ml of DCM. All the organic layers were combined and washed with 100 ml of purified water. The organic and aqueous layers were separated and the organic layer was dried over anhydrous sodium sulphate. The solvents were distilled completely at about 35-40°C under vacuum. To the residue, 100 ml of petroleum ether was added and the resulting mixture was stirred for 1hr at about 25-30°C under nitrogen atm. A solid separated and was filtered under nitrogen atmosphere and the filtered solid was washed with 50 ml of petroleum ether. The solid obtained was dried at about 40°C under vacuum for about 6 hours to afford 30 g of the title compound. % Yield = 82 % w/w. Purity by HPLC: 99.8 % All known and Unknown impurities = below 0.1 %.
SOR [α] 20 : -37.79° (as such) (C= 1% in EtOH at 589nm); -38.92° (On anhydrous basis) MASS (M/S): 369.2; Assay by HPLC (%w/w) : 31.85; Content of PTSA (%w/w by titrimetry) : 30.47.

### EXAMPLE 3: PREPARATION OF PERINDOPRIL TOSYLATE USING ETHANOL

Perindopril free acid (15 g) obtained from example 1 and para toluene sulfonic acid (9.38 g) and ethanol were charged into a clean and dry 4 neck RBF followed by stirring for about 3-4 hrs. The solvent was distilled completely at about 40-45°C under vacuum. 30 ml of purified water and 75 ml of DCM were charged and the resulting mixture was stirred for 25-30 min. The organic and aqueous layers were separated and the aqueous layer was extracted with 45 ml of DCM. The organic layers were combined and washed with 30 ml of purified water. The organic and aqueous layers were separated and the organic layer was dried over anhydrous sodium sulphate. The solvent was distilled completely at about 35-40°C under vacuum. Petroleum ether (75 ml) was charged and this mixture was stirred at 25-30°C for about 1hr under a nitrogen atmosphere. A solid separated and was filtered under nitrogen and the solid was washed with 30 ml of petroleum ether under nitrogen. The solid obtained was dried at about 40°C under vacuum for about 6 hrs to afford 18.2 g of the title compound. %Yield = 83 % w/w. Purity by HPLC: 99.8 %; All known & Unknown impurities = below 0.1 %. SOR [α] 20 : -35.87° (as such) (C= 1% in EtOH at 589nm); -37.04° (On anhydrous basis) MASS (M/S): 369.2; Assay by HPLC (%w/w) : 32%w/w; Content of PTSA (%w/w by titrimetry) : 30.29 %w/w.

**Table-1: ¹H NMR Assignments of the Perindopril tosylate salt**

| **Spectrum No.** | **Chemical shift (δ ppm)** | **Peak multiplicity** | **Number Assigned to the Proton** |
|---|---|---|---|
| 1 | 0.892-.-0.915 | 3 H (m) | 16 |
| 2 | 1.105-1.271 | 8 H (m) | 18, 11 & 15 |
| 3 | 1.614-2.221 | 14 H (m) | 1, 2, 3, 4, 5, 6, 7 & 14 |
| 4 | 4.239 | 2 H (q) | 17 |
| 5 | 2.288 | 1 H (s) | 25 |
| 6 | 7.098-7.481 | 4 H (m) | 20, 21, 23 & 24 |
| 7 | 9.359 | 1 H (NH. b) | 12 |
| 8 | 12.542 | 1 H (COOH, b) | 9 |

**Table-2: FTIR Band Assignments of the Perindopril tosylate salt**

| **Spectrum No.** | **Observed Band Frequency (cm⁻¹)** | **Assignment** |
|---|---|---|
| 1 | 816.1 | S-O stretching |
| 2 | 1140.6 | S=O stretching |
| 3 | 1219.5 | C-N stretching |
| 4 | 1383.6 | CH₃ bending |
| 5 | 1560.3 | N-H (bending) |
| 6 | 1742.3 | C=O stretching (Ethyl ester) |
| 7 | 2860.1 | C-H stretching (CH₃-CH-NH) |
| 8 | 2931.3 | C-H stretching (NH-CH-propyl) |
| 9 | 3447.1 | O-H stretching (-COOH) |

### Example 4: Preparation Of Perindopril Tosylate

Perindopril erbumine (40 g) was partially dissolved in 80 ml water. The mixture was cooled to 10° C and diluted HCl (prepared from 10 ml HCl 32% and 10 ml water) was added dropwise. The solution was stirred for 30 min at 10° C, heated to 25°C and NaCl (18.67 g) was added. The mixture was stirred for 15 min and extracted with Toluene/DCM (96 ml/24 ml and 64ml/16 ml), the combined organic phases were dried over Na₂SO₄ (21 g) and DCM was removed by drying under reduced pressure. Then water (240 ml) and PTSA (16.82 g) were added and the resulting mixture was stirred for 3 hours at room temperature, forming a three-phase mixture. To the three phase mixture was added heptane (16 mL), forming a biphasic mixture. The aqueous phase was extracted with toluene/heptane (180 mL/18 mL) and with heptane (120 ml). The aqueous phase was spray dried to yield the title compound as white powder.

### Example 5: Preparation of Perindopril Tosylate

Perindopril erbumin (30 g) was dissolved in 60 ml water. The mixture was cooled to 10° C and HCl (32%, 7.5 mL) was added dropwise during 10-15 min. The resulting solution was stirred for 30 min at 10° C. The solution was then heated to 25° C and NaCl (13.96 g) was added. The mixture was stirred for 15 min and extracted with toluene/DCM (72 mL/18 ml and 48mL/12 mL), and the DCM was then removed under reduced pressure. The resulting mixture was filtered under vacuum for salts removal. Then water (180 mL) and PTSA (11.88 g) were added to the filtrate, and the resulting mixture was stirred for 3 hours at room temperature to form a three phase mixture. To the three-phase mixture was added heptane (18 mL) to form a biphasic mixture. The biphasic was washed with toluene/heptane (135 mL/14 mL) and with heptane (90 ml). The aqueous phase was then dried in SD to yield the title compound as white powder.

### Example 6: Preparation of Perindopril Tosylate

Perindopril Erbumine (30 g) was dissolved in 90 ml water. The mixture was cooled to 10° C and dilute HCl (prepared from 7.6 mL HCl 32% and 7.6 mL) water) was added dropwise. The solution was stirred for 30 min at 10° C, then it was heated to 25°C and NaCl (20 g) was added. The resulting mixture was stirred for 15 min and extracted with DCM (90mL and 60 mL). Toluene (60 mL) was then added and the DCM was distilled. The resulting mixture was filtered over hyflo (10 g). Distilled water (180 mL) and PTSA (13 g) were added and the resulting mixture was stirred for 3 hours at room temperature to form a three phase mixture. Heptane was added to the three phase mixture, and the biphasic mixture formed thereby was washed with toluene/heptane (90 mL/18 mL) and then with heptane (90 mL). The aqueous phase was then dried in SD to yield the title compound as white powder;

### Example 7: Preparation of Perindopril Tosylate

A solution of PTSA (3.6g) in EtOAc (30 mL) was added dropwise to a turbid solution of Perindopril erbumine (3 g) in toluene (30 mL). The turbid solution turned clear and immediately turbid again. The solution was then stirred overnight at room temperature. A precipitate formed and was removed by suction filtration. The mother liqueur was then evaporated to yield a slightly yellow oil.

### Example 8: Preparation of Perindopril Tosylate

Perindopril erbumine (75 g) was dissolved in 225 ml water. The mixture was cooled to 10° C and dilute HCl (prepared from 19 mL HCl 32% and 19 mL water) was added water) dropwise. The resulting solution was stirred at 10° C for 30 min, then heated to 25° C and NaCl (50 g) was added. The resulting mixture was stirred for 15 min and extracted with DCM (225 mL and 60 mL). The aqeuoes phase was reextracted with DCM (150 mL) and the combined organic phase was evaporated to dryness. Toluene (225 mL), distilled water (450 mL) and PTSA (paratoluene sulphonic acid) (323 g) were added. The resulting mixture was stirred for 3 hours at room temperature. The toluene layer was separated and the aqueous phase was washed with toluene (225 mL) and hexane (250mLX2). The aqueous phase was lyophilized to yield the title compound as a white powder.

### Example 9: Preparation of Perindopril Tosylate

Perindopril erbumine (15 g) was dissolved in water (30 mL). The mixture was cooled to 10° C and dilute HCl (prepared from 7.6mL HCl 32% and 7.6 mL water) was added dropwise. The resulting solution was stirred at 10° C for 40 min. EtOAc (100 mL) was added to form a 2-phase mixture, and the layers were separated. NaCl and EtOAC were added to the aqeous phase and the layers were separated. PTSA (6.75 g) was added to the combined organic phase and the resulting mixture was stirred for 3 hours at room temperature. The solvent was then removed in SD (spray drying) to yield the title compound as a white powder.

### Example 10: Preparation of Perindopril Tosylate

The aqueous phase obtained in example 4 was mixed with lactose monohydrate (19.47g) and water (370 mL for dissolution) was added, and the solution was then dried in SD (spray drying).

### Example 11: Preparation of Perindopril Tosylate

The aqueous phase obtained in example 6 was mixed with NaHCO₃ (0.28 g) and water (90 mL for dissolution) was added and the solution was dried in SD (spray drying).

### Example 12: Preparation of Perindopril Tosylate

Perindopril free acid (50gm) was suspended in water (150ml) in a 500ml three neck RB flask at 25-30°C, p-toluene sulfonic acid (27.7gm) was added and stirred to get clear solution. The clear solution was washed with toluene (2 X 50ml) and hexane (2 X 50ml). The aqueous reaction mass was filtered though hyflo-supercel bed and washed with water (50ml). The filtered mass was cooled to -40°C and subjected to lyophilyzation. On lyophiolization, perindopril tosylate 68.5gm obtained as white powder.

### Example 13: Preparation of Perindopril Tosylate

Perindopril free acid (50gm) was suspended in water (150ml) in a 500ml three neck RB flask at 25-30°C, p-toluene sulfonic acid (27.7gm) was added and stirred to get clear solution. The aqueous reaction mass was filtered though hyflo-supercel bed and washed with water (50ml). The filtered mass was cooled to -40°C and subjected to lyophiolization. On lyophilyzation, perindopril tosylate 70gm obtained as white powder.

## Claims

1. Perindopril tosylate salt.

2. The perindopril tosylate of claim 1, in amorphous form.

3. The perindopril tosylate of claim 1 or claim 2, **characterized by** data selected from the group consisting of: a ¹H NMR spectrum substantially as illustrated in Figure 1; a proton NMR (D₂O-exchange) spectrum substantially as illustrated in Figure 2; a mass spectrum substantially as illustrated in Figure 3; a FTIR spectrum substantially as illustrated in Figure 4; a solid-state ¹³C NMR spectrum with signals at about 126.3, 128.4 and 168.3± 0.2 ppm; and a combination thereof.

4. The perindopril tosylate of claim 3, further **characterized by** data selected from the group consisting of: a solid-state ¹³C NMR spectrum having chemical shifts differences between the signal exhibiting the lowest chemical shift and another in the chemical shift range of 100 to 180 ppm of about 0.0, 2.1 and 42.0 ± 0.1 ppm; a ¹³C NMR spectrum substantially as illustrated in Figure 8; a solid-state ¹³C NMR spectrum substantially as illustrated in Figure 9; and a combination thereof.

5. The perindopril tosylate of any of claims 1 to 4, **characterized by** a X-ray powder diffraction pattern substantially as illustrated in Figure 5.

6. A pharmaceutical composition, comprising perindopril tosylate according to any of claims 1 to 5 and at least one pharmaceutically acceptable excipient.

7. Perindopril tosylate according to any of claims 1 to 5 for use in medicine.

8. Perindopril tosylate according to any of claims 1 to 5 for use in treating hypertension and/or heart failure.

9. A process for preparing perindopril tosylate according to any of claims 1 to 5 comprising:
(a) providing a solution of perindopril and paratoluene sulfonic acid in one or more reaction solvents;
(b) removing the reaction solvent(s) from the solution; and
(c) recovering the perindopril tosylate.

## Patentansprüche

1. Perindopriltosylatsalz.

2. Perindopriltosylat nach Anspruch 1 in amorpher Form.

3. Perindopriltosylat nach Anspruch 1 oder Anspruch 2, **gekennzeichnet durch** Daten, ausgewählt aus der Gruppe, bestehend aus einem ¹H-NMR-Spektrum, im Wesentlichen wie in Figur 1 dargestellt, einem Protonen-NMR- (D₂O-Austausch-) Spektrum, im Wesentlichen wie in Figur 2 dargestellt, einem Massenspektrum, im Wesentlichen wie in Figur 3 dargestellt, einem FTIR-Spektrum, im Wesentlichen wie in Figur 4 dargestellt ist, einem ¹³C-Festphasen-NMR-Spektrum mit Signalen bei ungefähr 126,3, 128,4 und 168,3 ± 0,2 ppm und einer Kombination davon.

4. Perindopriltosylat nach Anspruch 3, das weiter durch Daten gekennzeichnet ist, ausgewählt aus der Gruppe, bestehend aus einem ¹³C-Festphasen-NMR-Spektrum mit Differenzen der chemischen Verschiebungen zwischen dem Signal, das die geringste chemische Verschiebung zeigt, und einem anderen, im Bereich von einer chemischen Verschiebung von 100 bis 180 ppm von ungefähr 0,0, 2,1 und 42,0 ± 0,1 ppm, einem ¹³C-NMR-Spektrum, im Wesentlichen wie in Figur 8 dargestellt, einem ¹³C-Festphasen-NMR-Spektrum, im Wesentlichen wie in Figur 9 dargestellt, und einer Kombination davon.

5. Perindopriltosylat nach einem der Ansprüche 1 bis 4, **gekennzeichnet durch** ein Röntgenpulverdiagramm, im Wesentlichen wie in Figur 5 dargestellt.

6. Pharmazeutische Zusammensetzung, die Perindopriltosylat gemäß einem der Ansprüche 1 bis 5 und wenigstens einen pharmazeutisch verträglichen Hilfsstoff umfasst.

7. Perindopriltosylat gemäß einem der Ansprüche 1 bis 5 zur Verwendung in der Medizin.

8. Perindopriltosylat gemäß einem der Ansprüche 1 bis 5 zur Verwendung bei der Behandlung von Hochdruck und/oder Herzversagen.

9. Verfahren zur Herstellung von Perindopriltosylat gemäß einem der Ansprüche 1 bis 5, das Folgendes umfasst:
(a) Bereitstellen einer Lösung von Perindopril und Paratoluensulfonsäure in einem oder mehreren Reaktionslösungsmitteln,
(b) Entfernen des Reaktionslösungsmittels / der Reaktionslösungsmittel aus der Lösung und
(c) Wiedergewinnen des Perindopriltosylats.

## Revendications

1. Sel tosylate de périndopril.

2. Tosylate de périndopril selon la revendication 1 sous forme amorphe.

3. Tosylate de périndopril selon la revendication 1 ou la revendication 2 **caractérisé par** des données choisies dans le groupe consistant en : un spectre de RMN de ¹H sensiblement tel qu'illustré dans la figure 1 ; un spectre de RMN du proton (échange de D₂O) sensiblement tel qu'illustré dans la figure 2 ; un spectre de masse sensiblement tel qu'illustré dans la figure 3 ; un spectre de FITR sensiblement tel qu'illustré dans la figure 4 ; un spectre de RMN de ¹³C à l'état solide avec des signaux à environ 126,3, 128,4 et 168,3 ± 0,2 ppm ; et une combinaison de ceux-ci.

4. Tosylate de périndopril selon la revendication 3 **caractérisé en outre par** des données choisies dans le groupe consistant en : un spectre de RMN de ¹³C à l'état solide ayant des différences de déplacement chimique entre le signal présentant le plus bas déplacement chimique et un autre dans la plage de déplacement chimique de 100 à 180 ppm d'environ 0,0, 2,1 et 42,0 ± 0,1 ppm ; un spectre de RMN de ¹³C sensiblement tel qu'illustré dans la figure 8 ; un spectre de RMN de ¹³C à l'état solide sensiblement tel qu'illustré dans la figure 9 ; et une combinaison de ceux-ci.

5. Tosylate de périndopril selon l'une quelconque des revendications 1 à 4 **caractérisé par** un diagramme de diffraction des rayons X de poudre sensiblement tel qu'illustré dans la figure 5.

6. Composition pharmaceutique comprenant le tosylate de périndopril selon l'une quelconque des revendications 1 à 5 et au moins un excipient pharmaceutiquement acceptable.

7. Tosylate de périndopril selon l'une quelconque des revendications 1 à 5 destiné à être utilisé en médecine.

8. Tosylate de périndopril selon l'une quelconque des revendications 1 à 5 destiné à être utilisé dans le traitement de l'hypertension et/ou de l'insuffisance cardiaque.

9. Procédé pour préparer le tosylate de périndopril selon l'une quelconque des revendications 1 à 5 comprenant :
(a) la fourniture d'une solution de périndopril et d'acide paratoluènesulfonique dans un ou plusieurs solvants réactionnels ;
(b) le retrait du ou des solvants réactionnels de la solution ; et
(c) la récupération du tosylate de périndopril.
